# EUROPEAN PATENT APPLICATION

(11) **EP 4 850 611 A1**
(43) Date of publication of application: **09.12.2026**
(21) Application number: 25180557.8
(22) Date of filing: 03.06.2025
(51) Int. Cl.: A61B 8/08, A61B 8/00

(54) **CLASSIFYING KINETIC FUNCTION**

(71) Applicant: Koninklijke Philips N.V., 5656 AE Eindhoven (NL)
(72) Inventor: GERI, Oren, Eindhoven (NL); SEMO, Sarit, Eindhoven (NL); ANKRI, Yanai Victor, 5656AG Eindhoven (NL); BECHER, Menachem, Eindhoven (NL); SADIKOV, Alex, Eindhoven (NL); LOTRINGER, Yonit, Eindhvoen (NL); RAZ, Eliyahu, Eindhoven (NL); GOSHEN GOLDSHINE, Elad, Eindhoven (NL); YAACOBI, Marina, Eindhoven (NL)
(74) Representative: Philips Intellectual Property & Standards

(57) **Abstract**

Proposed concepts thus aim to provide schemes, solutions, concepts, designs, methods and systems pertaining to classifying kinetic function of a heart. In particular, embodiments aim to provide a method for classifying kinetic function of a heart by segmenting the heart in multiple ultrasound images showing the heart over time so that the movement of a ventricle of the heart can be determined and then classified as normal or abnormal, thus providing an automated and standardised way of assessing the kinetic function of a heart.

## Description

### FIELD OF THE INVENTION

This invention relates to the field of classifying kinetic function of a heart.

### BACKGROUND OF THE INVENTION

Left ventricle (LV) segmental wall motion (SWM) is one of the imaging markers used to evaluate the LV function and therefore to recognize ischemic heart disease by echocardiography. It involves classifying each segment of the ventricle's 17 segments with a score indicative of its kinetic function, which refers to the motion and contraction performance of the specific portion of the ventricular wall during the cardiac cycle.

In practice, LV SWM is evaluated qualitatively, by visual estimation, and requires a high level of training and expertise. It is limited by inter- and intra-observer variability, due to being evaluated by visually assessing segmental kinetics based on myocardial radial movement, and therefore is highly subjective. These problems equally apply to assessing the kinetic function of other parts of a heart.

### SUMMARY OF THE INVENTION

The invention is defined by the claims.

According to examples in accordance with an aspect of the invention, there is provided a method for classifying kinetic function of a heart.

The method comprises: obtaining a plurality of ultrasound images comprising a representation of at least part of a heart over a period of time; segmenting the at least part of the heart into at least one ventricle in each of the plurality of ultrasound images; determining the contours of the at least one ventricle in each of the plurality of ultrasound images; and classifying the at least one ventricle with one of a plurality of classifications indicating kinetic function of the heart based on movement of the determined contours of the at least one ventricle respectively, wherein the plurality of classifications comprise normal and abnormal.

Proposed concepts thus aim to provide schemes, solutions, concepts, designs, methods and systems pertaining to classifying kinetic function of a heart. In particular, embodiments aim to provide a method for classifying kinetic function of a heart by segmenting the heart in multiple ultrasound images showing the heart over time so that the movement of a ventricle of the heart can be determined and then classified as normal or abnormal, thus providing an automated and standardised way of assessing the kinetic function of a heart.

In other words, an automated method of performing an assessment of part of a heart's kinetic function (e.g., LV SWM) is provided, therefore facilitating the provision of an automatic SWM result for any echocardiography ultrasound exam, whether it is obtained during the exam, or as an offline retrospect evaluation. Furthermore, the assessment of the part of the heart's (e.g., the LV's) kinetic function will be more reliable and accurate than a user's mere visual assessment. This also enables untrained medical professionals to obtain a complete LV function assessment without the need for an expert to be present, as well as shortening the examination time even if an expert is present.

Ultimately, an improved method for classifying kinetic function of a heart is provided.

In some embodiments, the plurality of classifications may comprise at least one of hypokinetic and/or akinetic. Specifying that an abnormal ventricle/segment of the heart is hypokinetic or akinetic provides additional clinically relevant information to a user.

In some embodiments, the plurality of ultrasound images may comprise multiple views of the at least part of the heart. This makes the assessment of the heart's movement more reliable, resulting in more reliable classifications, while also allowing for the classification of territories which are typically spread across multiple views.

In some embodiments, the multiple views may comprise two-chamber, three-chamber, and four-chamber views. These views have been found to be particularly useful for the assessment of the heart's movement.

In some embodiments, segmenting the at least part of the heart may comprise using an automated border detection algorithm. This has been found to be a particularly effective method for segmenting the at least part of the heart.

In some embodiments, classifying the at least one ventricle may comprise: determining at least one numerical feature representing movement of the determined contours of the ventricle; and classifying the ventricle with one of the plurality of classifications based on the determined at least one numerical feature of said ventricle. This provides an efficient and reliable way to classify the movement of the one or more ventricles.

In some embodiments, the at least one numerical feature may comprise a ratio between minimum and maximum positions of the determined contours of said ventricle over the period of time. This has been found to be a particularly effective numerical feature on which to base the classification of the ventricle's movement.

In some embodiments, the at least one numerical feature may comprise global longitudinal strain of the heart. This has also been found to be a particularly effective numerical feature to aid in the classification of the ventricle's movement.

In some embodiments, the plurality of ultrasound images may comprise multiple views of the at least part of the heart, a plurality of numerical features may be determined representing movement of the determined contours of the ventricle, and the method may further comprise: classifying at least one territory of the heart present in the plurality of ultrasound images with one of the plurality of classifications based on the classification of the ventricle and the plurality of numerical features. This allows the kinetic function of the heart to be analysed on a territorial level.

In some embodiments, the method may further comprise classifying at least one segment of the at least one territory with one of the plurality of classifications based on the classification of the ventricle, the classification of the at least one territory, and the plurality of numerical features. This allows the kinetic function of the heart to be analysed on a segmental level.

In some embodiments, classifying the at least one ventricle may comprise using a first linear discriminant analysis classifier to classify between normal and abnormal. This has been found to be a particularly effective form of classifier for classifying the heart's movement.

In some embodiments, classifying the at least one ventricle may further comprise using a second linear discriminant analysis classifier to classify between hypokinetic and akinetic. It has been found that using a second, separate linear discriminant analysis classifier to further discriminate between hypokinetic and akinetic (both forms of abnormal movement) allows both classifiers to be more efficient and reliable in their assessments.

In some embodiments, the method may further comprise determining a kinetic function score for the heart based on the classification of each of the at least one ventricle and generating an output comprising the kinetic function score. This allows a user to be efficiently informed as to the overall status of the heart's kinetic function.

In some embodiments, the at least one ventricle may comprise a plurality of segments, and classifying the at least one ventricle may comprise: if the at least one ventricle is classified as abnormal: determining the contours of each of the plurality of segments in each of the plurality of ultrasound images; and classifying each of the plurality of segments with one of the plurality of classifications based on movement of the determined contours of each of the plurality of segments respectively. This allows the ventricle to be more closely studied only if its kinetic function appears to be abnormal, thus making the method more efficient at localising any potential issues with the kinetic function of the heart.

In some embodiments, the method may further comprise: determining the contours of each of one or more territories of the heart present in the plurality of ultrasound images; and classifying each of the plurality of territories with one of the plurality of classifications based on movement of the determined contours of each of the one or more territories respectively. This allows the kinetic function of the heart to be analysed on a more specific level without being as granular as classifying the heart per segment.

According to another aspect of the invention, there is provided a computer program comprising code means for implementing the method of any herein disclosed method when said program is run on a processing system.

According to another aspect of the invention, there is provided a system for classifying kinetic function of a heart. The system comprising a processing unit, wherein the processing unit is configured to: obtain a plurality of ultrasound images comprising a representation of at least part of a heart over a period of time; segment the at least part of the heart into at least one ventricle in each of the plurality of ultrasound images; determine the contours of the at least one ventricle in each of the plurality of ultrasound images; and classify the at least one ventricle with one of a plurality of classifications indicating kinetic function of the heart based on movement of the determined contours of the at least one ventricle respectively, wherein the plurality of classifications comprise normal and abnormal.

Thus, there may be proposed concepts for classifying kinetic function of a heart, and this may be done based on analysing the movement of contours of at least one segmented ventricle of the heart.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flow diagram of a method for classifying kinetic function of a heart according to a proposed embodiment;
Fig. 2A is a diagram showing how the movement of a segment can be assessed;
Fig. 2B is a graph showing how the length of a line in Fig. 2A varies over time;
Fig. 3 is a flow diagram of a method for classifying kinetic function of a heart according to a proposed embodiment;
Fig. 4 is a flow diagram of a method for classifying kinetic function of a heart according to a proposed embodiment;
Fig. 5 is a simplified block diagram of a system for classifying kinetic function of a heart according to a proposed embodiment; and
Fig. 6 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to classifying kinetic function of a heart. According to proposed concepts, a number of possible solutions may be implemented separately or jointly. That is, although these possible solutions may be described below separately, two or more of these possible solutions may be implemented in one combination or another.

Embodiments of the invention aim to provide a method for classifying kinetic function of a heart. This can be achieved by segmenting the heart in multiple ultrasound images showing the heart over time so that the movement of a ventricle of the heart can be determined and then classified as normal or abnormal, thus providing an automated and standardised way of assessing the kinetic function of a heart.

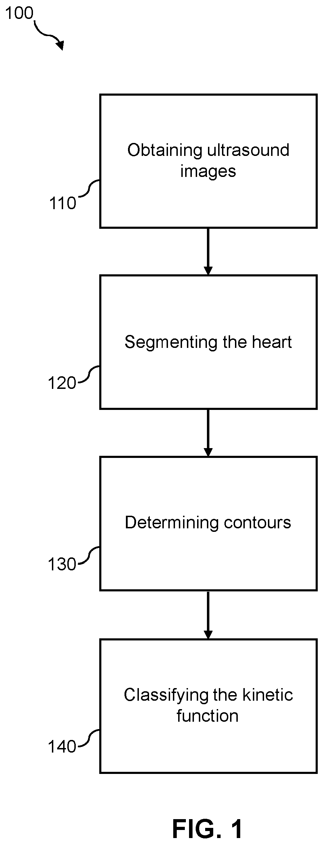

Referring now to Fig. 1, there is depicted a simplified flow diagram of a computer-implemented method 100 for classifying kinetic function of a heart according to a proposed embodiment.

The method 100 begins with the step 110 of obtaining a plurality of ultrasound images comprising a representation of at least part of a heart over a period of time. In other words, step 110 comprises obtaining (e.g., receiving, capturing, retrieving, etc.) two or more ultrasound images showing at least a portion of a (subject's) heart over a period of time, i.e., the two or more ultrasound images are from two distinct points in time (and preferably within the same cardiac cycle / heartbeat).

In all embodiments, the at least part/portion of the heart must comprise at least part of a ventricle of the heart. The present invention is of particular use when applied to the left ventricle, therefore facilitating the recognition of ischemic heart disease.

Furthermore, in this embodiment, the plurality of ultrasound images comprise multiple views of the at least part of the heart (i.e., the ventricle), and in particular, two-chamber, three-chamber, and four-chamber views. These views have been found to be particularly useful for the assessment of the heart's movement and also allow for the classification of territories (which are typically spread across multiple views) though in other embodiments, other standard and/or non-standard views can be used or even merely a single view. In this embodiment, all of the plurality of ultrasound images are processed together (i.e., in steps 120, 130 and 140) but it should be noted that in other embodiments, the remainder of the method can be performed separately for each set of images of each view respectively.

Step 120 comprises segmenting the at least part of the heart into at least one ventricle in each of the plurality of ultrasound images, i.e., dividing the (at least a) portion of the heart shown in the ultrasound images into one or more ventricles in each of the said ultrasound images. Step 120 thus comprises finding at least one ventricle in each of the ultrasound images and segmenting each image to discriminate the (at least one) ventricle from the rest of the image respectively.

Step 130 comprises determining the contours of the at least one ventricle in each of the plurality of ultrasound images. Essentially, in this step, after dividing the representation of the heart into one or more ventricles, the contours (e.g., boundaries) of each ventricle are identified and outlined. Step 130 can thus alternatively be understood as 'extracting contours for each identified ventricle in the plurality of ultrasound images' or 'determining region boundaries for each segmented ventricle in each of the plurality of ultrasound images'.

In this embodiment, steps 120 and 130 are facilitated by using an automated border detection algorithm (to essentially perform both segmentation and determining of contours in a single step). This has been found to be a particularly effective method for segmenting (and determining the contours of) the at least part of the heart, though as the skilled person would appreciate, in other embodiments, steps 120 and/or 130 can be performed in any suitable way (e.g., in a single joint step or separately).

Finally, step 140 comprises classifying the at least one ventricle with one of a plurality of classifications (the plurality of classifications comprising at least normal and abnormal) based on movement of the determined contours of the at least one ventricle respectively. For example, if the heart has been segmented into two ventricles and the contours of each ventricle determined in each of the ultrasound images (which show the heart over a period of time), then based on how the contours change/move for each respective ventricle across the ultrasound images, each ventricle can be classified as normal or abnormal meaning that its kinetic function is normal or abnormal respectively. For instance, how the contours change over time across the ultrasound images can be determined as a time-varying signal which is then analysed to classify the kinetic function of the ventricle - for example, if the time-varying signal does not vary by more than a first predetermined threshold or varies by more than a second predetermined threshold, then the ventricle can be classified as abnormal (e.g., akinetic or hypokinetic respectively).

In this embodiment, classifying the at least one ventricle comprises using a first linear discriminant analysis (LDA) classifier to classify between normal and abnormal. This has been found to be a particularly effective form of classifier for classifying the heart's movement though, as the skilled person would appreciate, in other embodiments, a different type of classifier or algorithm can be used to classify between normal and abnormal kinetic function.

In this embodiment, the plurality of classifications further comprise both hypokinetic and akinetic (though in other embodiments it may include only one or none of these classifications). That is, in this embodiment, the abnormal classification essentially branches into hypokinetic and akinetic as these are both abnormal classifications of kinetic function of a ventricle. Accordingly, in this embodiment, classifying the at least one ventricle in step 140 further comprises using a second LDA classifier to classify between hypokinetic and akinetic (i.e., to further classify the ventricle after it has already been classified as abnormal by the first LDA classifier). It has been found that using a second, separate linear discriminant analysis classifier to further discriminate between hypokinetic and akinetic (both forms of abnormal movement) allows both classifiers to be more efficient and reliable in their assessments. Of course, if the ventricle is found by the first LDA classifier to be normal then the second LDA classifier would not be used to further classify the ventricle. Furthermore, if the plurality of classifications does not comprise akinetic and/or hypokinetic then there is no need for the second LDA classifier.

In summary, this invention discloses a methodology in which cardiac ultrasound clips (i.e., a collection of frames over time) can be analysed to directly classify the kinetic function of ventricles (as well as territories and/or segments) of a heart. In a specific example of the invention, a locked machine learning algorithm which utilizes a set of Linear Discriminant Analysis (LDA) classifiers can be used to do so. In this specific example, the first step is to acquire a series of images (in the form of a steam of data or a video clip) from all three apical views of the heart (namely, 2-chamber, 3-chamber, and 4-chamber). The next step is to apply an automated border detection algorithm to obtain the contour of the left ventricle of the heart as a function of time for each apical view. From these contours, the resulting ventricle ejection fraction (EF) and strain is calculated. Based on the obtained contours, a series of numerical features are then calculated representing the segmental movement for the triplet of apical views. The numerical features are then input into a set of hierarchical pre-trained linear classifiers to receive classifications of the LV function on several levels: a global level (indicating kinetic function of the entire ventricle); a territorial level (indicating kinetic function of a region within the ventricle fed by a specific coronary artery); and a segmental level (indicating kinetic function of a specific segment of the ventricle). Each level's output is a classification on a scale of 1: Normal; 2: Hypokinetic; or 3: Akinetic. Each level output can also serve as an additional input(s) to the next level (i.e., the level below). In addition, the global EF and strain for each apical view can be input into the first (global) level.

Finally, the algorithm can calculate the apex score as the average of the scores from the four apical segments and determine the wall motion score index (WMSI) as the average of the segments, excluding the apex. Segment classifications can be presented separately for each of the plurality of views and can be visually displayed, for example, on a bull's eye diagram.

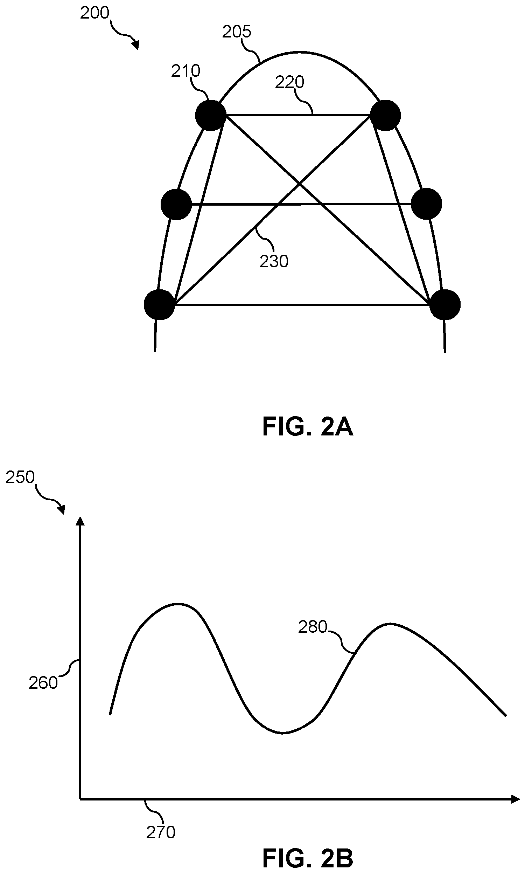

Referring now to Fig. 2A, there is depicted a diagram 200 showing how the movement of a segment can be assessed. In particular, diagram 200 depicts a seven lines model, with the rounded shape 205 representing a contour of a ventricle, in this case, the ventricle inner boundary (in a specific ultrasound image/frame). The contour 205, i.e., the ventricle, is divided into six segments, three on each side of the apex (the topmost point of the contour 205). The points 210 are selected as the central point of each segment and is taken to represent it. Seven lines, for example 220 and 230, then connect the central points, as shown, and can be measured in each image/frame to thereby analyse how the contour 205 moves over time as the contour moving results in the lengths of the lines, e.g., 220 and 230 changing.

For instance, now referring to Fig. 2B, there is depicted a graph 250 showing the length of line 230 changes/varies over time as the ventricle, and therefore the contour 205 of said ventricle, moves. Axis 260 represents length/distance of line 230 and axis 270 represents time. The line 280 thus shows how the length of line 230 varies over time as the contour 205 moves due to the ventricle contracting and expanding due to the cardiac cycle.

A ratio can then be calculated between the minimum and maximum length/distance values of line 230 (over a single heart beat), resulting in a single number being able to represent (at least an aspect/part of) the movement of the contour 205.

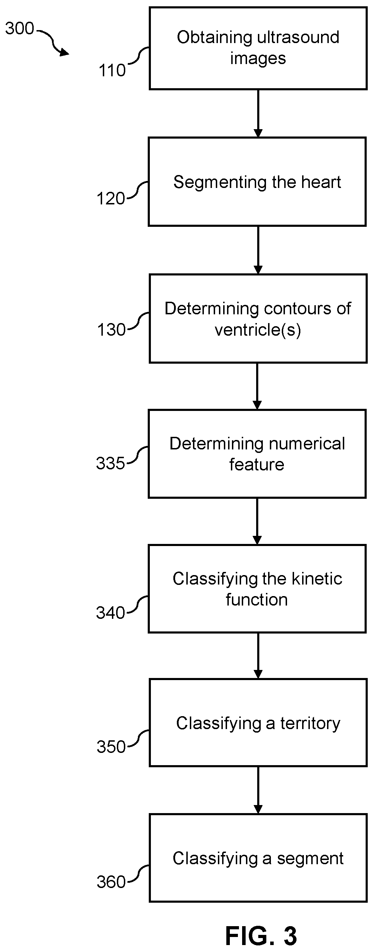

Referring now to Fig. 3, there is depicted a flow diagram of a computer-implemented method 300 for classifying kinetic function of a heart according to a proposed embodiment. Steps 110, 120, and 130 are substantially the same as have been described in relation to the method 100 in Fig. 1.

In this embodiment, step 140 of method 100 has essentially been replaced with steps 335 and 340. Step 335 comprises determining a plurality of numerical features representing movement of the determined contours of the ventricle (though in other embodiments only one numerical feature may be determined). For instance, in this embodiment, the numerical features comprise a ratio between minimum and maximum positions of the determined contours of said ventricle over the period of time - this can be understood as a ratio or ratios between minimum and maximum positions of points on the predetermined contours of said ventricle over the period of time, as described above in relation to Fig. 2B. In other words, the ratio is between minimum and maximum lengths of lines connecting at least two predetermined points (such as central points of segments of the ventricle) on the contours of said ventricle over the period of time (which the plurality of ultrasound images represent). To be clear, a ratio between minimum and maximum positions of the determined contours of said ventricle over the period of time can be understood as a ratio between a minimum and a maximum length of a line connecting two specific/predetermined points on the determined contours of said ventricle over a period of time.

This provides an efficient and reliable way to classify the movement of the one or more ventricles, though in other embodiments, the one or more numerical features can be any suitable numerical features for representing movement of the determined contours of the ventricle, as the skilled person would appreciate. For example, in other embodiments, the at least one numerical feature can alternatively/additionally comprise global longitudinal strain of (a ventricle of) the heart. As the skilled person would know, global longitudinal strain (GLS) of a ventricle refers to the average percentage of deformation (shortening) along the length of the heart muscle within a specific coronary-supplied region during systole, indicating how well that region contracts. The skilled person would be aware of how this numerical feature could be determined from ultrasound images.

In step 340, the ventricle is classified based on the determined one or more numerical features of the one or more ventricles, thus providing an efficient and reliable way to classify the movement of said one or more ventricles. For instance, if the numerical feature for a first ventricle is above a first predetermined threshold or below a second predetermined threshold, the first ventricle can be classified as abnormal, and otherwise, it can be classified as normal.

In an example of the invention, the ratio described in relation to Fig. 2B is used as the numerical feature(s) in step 335, i.e., a set of numerical ratios between minimum and maximum values of time dependent signals, however, in other examples of the invention, other implementations of numerical features representing the kinetics of at least part of the heart could replace them.

Step 350 comprises classifying at least one territory of the heart present in the plurality of ultrasound images (which in this embodiment, comprise multiple views) with one of the plurality of classifications based on the classification of the ventricle and the plurality of numerical features. As the skilled person would know, a territory typically refers to a region of the heart muscle (myocardium) supplied by a specific coronary artery. As an example, when considering the 17-segment model of the left ventricle, a territory refers to the group of segments supplied by a specific coronary artery-usually the left anterior descending (LAD), right coronary artery (RCA), or left circumflex (LCx). In the 17-segment model of the left ventricle, there are therefore three territories, each containing a plurality of segments. The skilled person would be aware of the myriad ways in which a territory could be classified using one or more ventricle classifications and the plurality of numerical features indicating the movement of said one or more ventricles.

Step 360 then comprises classifying at least one segment of the at least one territory with one of the plurality of classifications based on the classification of the ventricle, the classification of the at least one territory, and the plurality of numerical features. As the skilled person would know, a segment typically refers to an anatomically or functionally distinct portion of the myocardium as defined by standardized cardiac imaging models. As an example, in the 17-segment model of the left ventricle, each segment represents a discrete unit of myocardial tissue that can be independently assessed for perfusion, wall motion, or viability. Each segment is assigned a specific spatial orientation (e.g., basal, mid, apical) and circumferential location (e.g., anterior, lateral, inferior), allowing reproducible analysis across imaging modalities. Accordingly, a plurality of such segments are grouped into coronary territories based on their predominant vascular supply. Again, the skilled person would be aware of the myriad ways in which a segment could be classified using one or more ventricle and territory classifications and the plurality of numerical features indicating the movement of said one or more ventricles.

In an alternative embodiment of the method 300, after step 340, if the at least one ventricle is classified as abnormal in step 340 (e.g., akinetic or hypokinetic), the method continues to a step of determining the contours of each of a plurality of segments of the ventricle (i.e., which the ventricle comprises) in each of the plurality of ultrasound images, followed by another step comprising classifying each of the plurality of segments with one of the plurality of classifications based on movement of the determined contours of each of the plurality of segments respectively (in much the same way as in step 140 or steps 335 and 340).

Alternatively, or in addition (to the consideration of segments), if the plurality of ultrasound images comprise multiple views, then the contours of one or more territories of the ventricle can be determined (which are typically spread across multiple views of a ventricle). Each of the one or more territories can then be classified based on movement of the determined contours of the one or more territories respectively (in much the same way as in step 140 or steps 335 and 340) using data included in the multiple views of the plurality of ultrasound images.

It should be noted that all herein features/descriptions relating to how the ventricles can be segmented, have their contours determined, have numerical features determined representing their movement, and be classified apply *mutatis mutandis* to the segments and territories (of said ventricles).

In a more specific example of the invention, the input borders for each apical view (two-chamber, three-chamber, and four-chamber) are used to extract features based on the seven-lines model (shown in Fig. 2A). As shown, a representative point is defined for each segment in every frame, and these points are connected by seven lines. The length of these lines are then used to calculate a 'distance ratio' defined as the minimal distance divided by the maximal distance over the duration of one heartbeat. Additionally, the Global Longitudinal Strain (GLS) can be included as a numerical feature. These features serve as inputs to a series of hierarchical LDA classifiers. A global classifier can determine whether, for example, the global left ventricular (LV) function is normal or abnormal. If the LV function is classified as globally normal, the wall motion score for all segments can be assigned a value of 1 (i.e., normal). If the LV function is found to be reduced (i.e., abnormal), the classification process continues to territorial and segmental classifiers. For segmental classification, a two-stage LDA classifier is used - segments classified as abnormal are then further classified as either hypokinetic or akinetic. Finally, the wall motion score index (WMSI) is calculated as the average score of 16 segments (e.g., from 1 to 3, as described above), excluding the apex. The skilled person would appreciate how the LDA classifiers could be pre-trained (for example, on a large dataset annotated by expert sonographers) and have their weights set for these purposes.

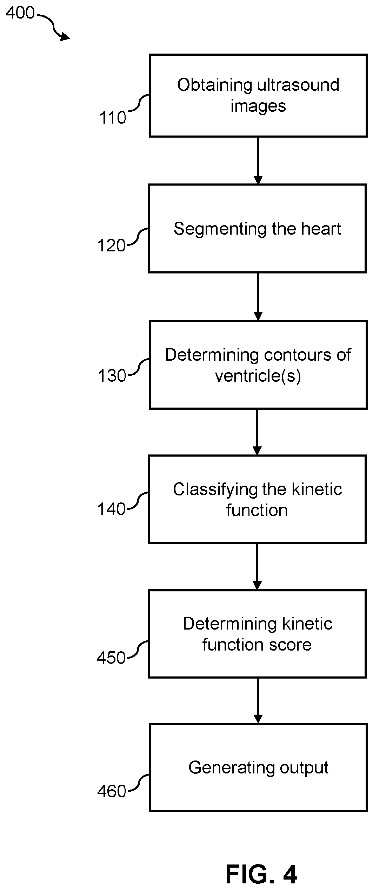

Referring now to Fig. 4, there is depicted a computer-implemented method 400 for classifying kinetic function of a heart according to a proposed embodiment. Steps 110, 120, 130 and 140 are substantially the same as those described in relation to method 100 of Fig. 1.

Step 450 comprises determining a kinetic function score for the heart based on the classification of each of the at least one ventricle (and/or segments, if the segments of the ventricle of the heart have been classified too - in other words, if the segments of a ventricle have been classified, then the scores of said ventricle's segments replace the score of said ventricle). For instance, the kinetic function score may take the form of a numerical score in which the higher it is, the more ventricles and/or segments have been classified as in some way abnormal. As the skilled person would be aware, there are many forms which the kinetic function score could take and thus many ways in which it could be determined - what is important is that the kinetic function score in some way indicates the classification(s) of each of the one or more ventricles and/or segments. Alternatively, or additionally, a kinetic function score for each of one or more territories of the ventricle can be determined based on the classification of each of the one or more territories (and/or segments, if the segments of the territory /territories have been classified too).

Step 460 comprises then generating an output comprising the kinetic function score, allowing a user to be efficiently informed as to the overall status of the heart's kinetic function. Generating an output comprising the kinetic function score can be understood as generating an output which facilitates communicating the kinetic function score to a user in any suitable way, e.g., via display on a screen or via an audio speaker, via signal/data to a software process, as an input to an AI model, etc.

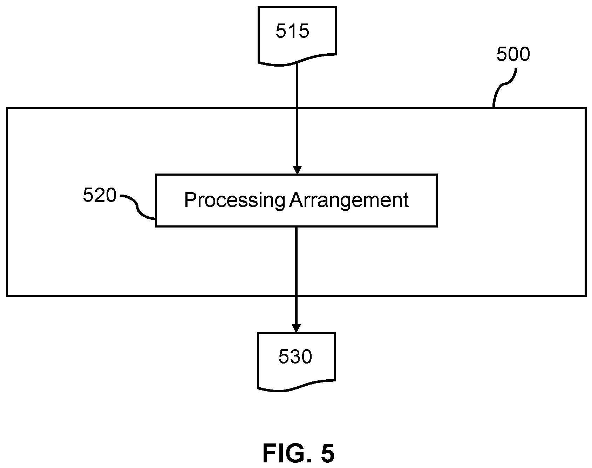

Referring now to Fig. 5, there is depicted a system 500 for classifying kinetic function of a heart according to a proposed embodiment. The system 500 comprises a processing arrangement 520.

The processing arrangement 520 can be configured to perform any of the herein-disclosed methods (e.g., method 100, 300, or 400), however, in this embodiment, the processing arrangement 520 is specifically configured to perform method 100, i.e., to: obtain a plurality of ultrasound images comprising a representation of at least part of a heart over a period of time; segment the at least part of the heart into at least one ventricle in each of the plurality of ultrasound images; determine the contours of the at least one ventricle in each of the plurality of ultrasound images; and classify the at least one ventricle with one of a plurality of classifications indicating kinetic function of the heart based on movement of the determined contours of the at least one ventricle respectively, wherein the plurality of classifications comprise normal and abnormal. In other words, the input 515 into the system is a plurality of ultrasound images comprising a representation of at least part of a heart over a period of time and the output 530 of the system comprises the classification of at least one ventricle of the at least part of the heart into one of a plurality of classifications indicating kinetic function of the heart.

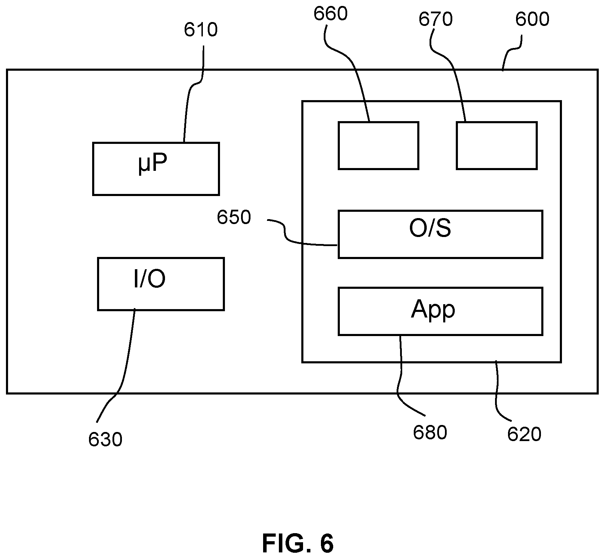

Fig. 6 illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620 and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor-based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at start-up, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1, 3 and 4, and the system of Fig. 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for classifying kinetic function of a heart, the method comprising:
obtaining a plurality of ultrasound images (110) comprising a representation of at least part of a heart over a period of time;
segmenting the at least part of the heart (120) into at least one ventricle in each of the plurality of ultrasound images;
determining the contours of the at least one ventricle (130) in each of the plurality of ultrasound images; and
classifying the at least one ventricle (140) with one of a plurality of classifications indicating kinetic function of the heart based on movement of the determined contours of the at least one ventricle respectively, wherein the plurality of classifications comprise normal and abnormal.

2. The computer-implemented method of claim 1 or 2, wherein the plurality of classifications comprises at least one of hypokinetic and/or akinetic.

3. The computer-implemented method of any preceding claim, wherein the plurality of ultrasound images comprise multiple views of the at least part of the heart.

4. The computer-implemented method of claim 3, wherein the multiple views comprise two-chamber, three-chamber, and four-chamber views.

5. The computer-implemented method of any preceding claim, wherein segmenting the at least part of the heart comprises using an automated border detection algorithm.

6. The computer-implemented method of any preceding claim wherein classifying the at least one ventricle comprises:
determining at least one numerical feature (335) representing movement of the determined contours of the ventricle; and
classifying the ventricle (340) with one of the plurality of classifications based on the determined at least one numerical feature of said ventricle.

7. The computer-implemented method of claim 6, wherein the at least one numerical feature comprises a ratio between minimum and maximum positions of the determined contours of said ventricle over the period of time.

8. The computer-implemented method of claim 6 or 7, wherein the at least one numerical feature comprises global longitudinal strain of the heart.

9. The computer-implemented method of any one of claims 6 to 8, wherein the plurality of ultrasound images comprise multiple views of the at least part of the heart, wherein a plurality of numerical features are determined representing movement of the determined contours of the ventricle, and wherein the method further comprises:
classifying at least one territory of the heart present in the plurality of ultrasound images with one of the plurality of classifications based on the classification of the ventricle and the plurality of numerical features.

10. The computer-implemented method of claim 9, further comprising classifying at least one segment of the at least one territory with one of the plurality of classifications based on the classification of the ventricle, the classification of the at least one territory, and the plurality of numerical features.

11. The computer-implemented method of any preceding claim wherein classifying the at least one ventricle comprises using a first linear discriminant analysis classifier to classify between normal and abnormal.

12. The computer-implemented method of claim 11, wherein classifying the at least one ventricle further comprises using a second linear discriminant analysis classifier to classify between hypokinetic and akinetic.

13. The computer-implemented method of any preceding claim wherein the method further comprises determining a kinetic function score for the heart (450) based on the classifications of each of the at least one ventricle and generating an output (460) comprising the kinetic function score.

14. A computer program comprising code means for implementing the computer-implemented method of any preceding claim when said program is run on a processing system.

15. A system (500) for classifying kinetic function of a heart, the system comprising:
a processing unit (520), wherein the processing unit is configured to:
obtain a plurality of ultrasound images comprising a representation of at least part of a heart over a period of time;
segment the at least part of the heart into at least one ventricle in each of the plurality of ultrasound images;
determine the contours of the at least one ventricle in each of the plurality of ultrasound images; and
classify the at least one ventricle with one of a plurality of classifications indicating kinetic function of the heart based on movement of the determined contours of the at least one ventricle respectively, wherein the plurality of classifications comprise normal and abnormal.
